# EUROPÄISCHE PATENTSCHRIFT

(11) **EP 3 723 707 B1**
(45) Veröffentlichungstag und Bekanntmachung des Hinweises auf die Patenterteilung: **16.09.2026**
(21) Anmeldenummer: 18814509.8
(22) Anmeldetag: 23.11.2018
(51) Int. Cl.: A61K 8/29, A61K 8/37, A61K 8/40, A61K 8/46, A61K 8/49, A61Q 17/04, C11D 11/00, C11D 3/00, C11D 17/00, C11D 3/34

(54) **SONNENSCHUTZMITTEL MIT REDUZIERTER NEIGUNG ZUR TEXTILVERFLECKUNG**
SUN PROTECTION WITH REDUCED INCLINATION FOR TEXTILE STAINING
PRODUITS SOLAIRES MOINS SUSCEPTIBLES DE TACHER LES TISSUS

(30) Priorität: 14.12.2017 DE 102017222739
(43) Veröffentlichungstag der Anmeldung: 21.10.2020
(73) Patentinhaber: Beiersdorf AG, 22529 Hamburg (DE)
(72) Erfinder: GUTZKE, Doreen, 29646 Bispingen (DE); BLECKMANN, Andreas, 22926 Ahrensburg (DE); NISSEN, Bente, 20144 Hamburg (DE); RASCHKE, Thomas, 25421 Pinneberg (DE)
(74) Vertreter: Beiersdorf AG
(86) Internationale Anmeldenummer: PCT/EP2018/082378
(87) Internationale Veröffentlichungsnummer: WO 2019/115206

(56) Entgegenhaltungen:
- EP-A1- 2 939 710
- EP-A2- 1 308 153
- WO-A1-2015/165715
- US-A1- 2007 025 939

## Beschreibung

Die vorliegende Erfindung betrifft Verfahren und Verwendungen zur Erleichterung der Auswaschbarkeit von UV-Lichtschutzfilter enthaltenden kosmetischen Zubereitungen aus Textilien, dadurch gekennzeichnet, dass dem Kosmetikum [(3Z)-3-[[4-[(Z)-[7,7-Dimethyl-2-oxo-1-(sulfomethyl)-3-bicyclo[2.2.1]heptanyliden]methyl]phenyl]methyliden]-7,7-dimethyl-2-oxo-1-bicyclo[2.2.1]heptanyl]methansulfonsäure (INCI: Terephthalylidene dicamphor sulfonic acid) und/oder dessen Natriumsalz zugesetzt wird.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter hervorgerufen. Neben den Verfärbungen durch UVA- und Breitbandfiltern tragen jedoch auch die UV-B-Filter Filter 2-Ethylhexyl 2-cyano-3,3-diphenyl-2-propenoat (INCI: Octocrylen) und/oder 2-Ethylhexyl 2-hydroxybenzoat (INCI: Ethylhexyl Salicylate) zur Verfärbung bei.

Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und Wege zu finden, um eine kosmetische Zubereitung (insbesondere ein Sonnenschutzmittel) enthaltend 2-Ethylhexyl 2-cyano-3,3-diphenyl-2-propenoat (INCI: Octocrylen) und/oder 2-Ethylhexyl 2-hydroxybenzoat (INCI: Ethylhexyl Salicylate) leichter aus den mit der Zubereitung kontaminierten Textilen herauswaschen zu können. Es sollte insbesondere die durch diese beiden UV-Filter hervorgerufene Textilverfleckung signifikant reduziert und entsprechende Verfleckungen leichter aus Textilien herausgewaschen werden.

Überraschend gelöst wird die Aufgabe durch Verwendungen gemäß einer der Ansprüche 1 und 2.

Die Begriffe kosmetische Zubereitung und Kosmetikum werden synonym verwendet.

Zwar sind dem Fachmann grundsätzlich kosmetische Sonnenschutzmittel enthaltend Terephthalylidene dicamphor sulfonic acid (auch bekannt unter dem Handelsnamen Mexoryl SX) sowie Octocrylen und/oder Ethylhexylsalicylat bekannt. Beispiele für derartigen Stand der Technik stellen die EP 2 939 710 A1, WO 2015/165715 A1, US 2007/0025939 A1 und EP 1 308 153 A2 dar. Dennoch konnten derartige Offenbarungen nicht den Weg zur vorliegenden Erfindung weisen, da die Wirkung der [(3*Z*)-3-[[4-[(*Z*)-[7,7-Dimethyl-2-oxo-1-(sulfomethyl)-3-bicyclo[2.2.1]heptanyliden]methyl]phenyl] methyliden]-7,7-dimethyl-2-oxo-1-bicyclo[2.2.1]heptanyl]methansulfonsäure beziehungsweise dessen Natriumsalzes auf die Auswaschbarkeit nicht erkannt wurde. Es wurde immer nur die Wirkung auf und für die Haut untersucht, während die Wirkung auf den "Kollateralschaden" der verschmutzen Textilen nie Gegenstand der Forschung gewesen ist. Diese Wirkung war umso überraschender, da die unterschiedlichen UV-Filter Terephthalylidendicamphersulfonsäure (Terephthalylidene dicamphor sulfonic acid) einerseits und Octocrylen und Ethylhexylsalicylat andererseits in der kosmetischen Zubereitung in unterschiedlichen Phasen vorliegen und besondere oberflächenaktive/ emulgierende Eigenschaften der Terephthalylidendicamphersulfonsäure nie beobachtet wurden, obwohl dieser UV-Filter seit Jahren in Emulsionen eingesetzt wird.

Es ist erfindungsgemäß von Vorteil, wenn die erfindungsgemäße kosmetische Zubereitung [(3*Z*)-3-[[4-[(*Z*)-[7,7-Dimethyl-2-oxo-1-(sulfomethyl)-3-bicyclo[2.2.1]heptanyliden]methyl] phenyl]methyliden]-7,7-dimethyl-2-oxo-1-bicyclo[2.2.1]heptanyl]methanesulfonsäure (INCI: Terephthalylidene dicamphor sulfonic acid) in einer Konzentration von 0,1 bis 10,0 Gewichts-%, bezogen auf das Gesamtgewicht der Zubereitung, enthält. Der erfindungsgemäß bevorzugte Einsatzbereich liegt dabei in einem Konzentrationsbereich von 0,1 bis 2,0 Gewichts-%, bezogen auf das Gesamtgewicht der Zubereitung.

Erfindungsgemäß können drei unterschiedliche Zubereitungssysteme unterschieden werden:
1. Zubereitungen die Octocrylen aber kein Ethylhexylsalicylat enthalten,
2. Zubereitungen die Ethylhexylsalicylat aber kein Octocrylen enthalten, sowie
3. Zubereitungen, die sowohl Octocrylen als auch Ethylhexylsalicylat enthalten.

Erfindungsgemäß besonders bevorzugt sind dabei Zubereitungen, die Ethylhexylsalicylat aber kein Octocrylen enthalten.

Die erfindungsgemäß vorteilhaften Ausführungsformen sind dabei dadurch gekennzeichnet, dass die kosmetische Zubereitung 2-Ethylhexyl 2-hydroxybenzoat (INCI: Ethylhexyl Salicylate) in einer Konzentration von 0,5 bis 5,0 Gewichts-%, bezogen auf das Gesamtgewicht der Zubereitung, enthält. Der erfindungsgemäß bevorzugte Einsatzbereich liegt dabei in einem Konzentrationsbereich von 2,0 bis 5,0 Gewichts-%, bezogen auf das Gesamtgewicht der Zubereitung.

Es ist erfindungsgemäß von Vorteil, wenn die kosmetische Zubereitung eine oder mehrere Verbindungen gewählt aus der Gruppe 4-(tert.-Butyl)-4'-methoxydibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält. Dabei ist insbesondere die Kombination mit 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) erfindungsgemäß bevorzugt.

Die erfindungsgemäß vorteilhafte Einsatzkonzentration für 4-(tert.-Butyl)-4'-methoxydiben-zoylmethan (INCI Butyl Methoxydibenzoylmethane) liegt bei 2,0 bis 5,0 Gewichts-%, bezogen auf das Gesamtgewicht der Zubereitung.

Die erfindungsgemäß vorteilhafte Einsatzkonzentration für (2-[-4-(Diethylamino)-2-hydroxybenzoyl] Benzoesäurehexylester (INCI: Diethylamino Hydroxybenzoyl Hexyl Benzoate) liegt bei 0,1 bis 10,0 Gewichts-%, bezogen auf das Gesamtgewicht der Zubereitung.

Die erfindungsgemäß vorteilhafte Einsatzkonzentration für 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) liegt bei 0,5 bis 5 ,0Gewichts-%, bezogen auf das Gesamtgewicht der Zubereitung.

Die erfindungsgemäße Zubereitung kann darüber hinaus erfindungsgemäß vorteilhaft weiter UV-Filter enthalten. So ist es insbesondere erfindungsgemäß von Vorteil, wenn die Zubereitung eine oder mehrere Verbindungen gewählt aus der Gruppe 2,4,6-Tris-[anilino-(p-carbo-2'-ethyl-1'-hexyloxy)]-1,3,5-triazin (INCI Ethylhexyl Triazone), 3,3,5-Trimethylcyclohexyl 2-hydroxybenzoat (INCI: Homosalate), Titandioxid und 2-Phenylbenzimidazol-5-sulfonsäuresalze (insbesondere das Natriumsalz) enthält.

Enthält die erfindungsgemäße Zubereitung 2,4,6-Tris-[anilino-(p-carbo-2'-ethyl-1'-hexyloxy)]-1,3,5-triazin (INCI Ethylhexyl Triazone), so ist es erfindungsgemäß vorteilhaft, dies in einer Konzentration von 0,5 bis 5,0 Gewichts-%, bezogen auf das Gesamtgewicht der Zubereitung, einzusetzen.

Enthält die erfindungsgemäße Zubereitung 3,3,5-Trimethylcyclohexyl 2-hydroxybenzoat (INCI: Homosalate), so ist es erfindungsgemäß vorteilhaft, dies in einer Konzentration von 2,0 bis 10,0 Gewichts-%, bezogen auf das Gesamtgewicht der Zubereitung, einzusetzen.

Enthält die erfindungsgemäße Zubereitung Titandioxid, so ist es erfindungsgemäß vorteilhaft, dies in einer Konzentration von 0,5 bis 5,0 Gewichts-%, bezogen auf das Gesamtgewicht der Zubereitung, einzusetzen.

Enthält die erfindungsgemäße Zubereitung 2-Phenylbenzimidazol-5-sulfonsäuresalze, so ist es erfindungsgemäß vorteilhaft, dies in einer Konzentration von 0,5 bis 3,0 Gewichts-%, bezogen auf das Gesamtgewicht der Zubereitung, einzusetzen.

Es sollte jedoch in jedem Falle auf den Einsatz von 3-(4-Methylbenzyliden)-campher, 2-Hydroxy-4-methoxybenzophenon (INCI: Oxybenzon), 4-Methoxyzimtsäureisoamylester und 4-Methoxyzimtsäureethylhexylester verzichtet werden.

Erfindungsgemäß vorteilhafte Ausführungsformen sind auch dadurch gekennzeichnet, dass die kosmetische Zubereitung eine oder mehrere der Verbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
   und/oder deren Alkalisalze und/oder
   1-Hydroxy-4-methyl-6-(2,4,4-trimethylpentyl)-2(1H)-pyridon (Pirocton) oder dessen Monoethanolamin-Salz
   enthält.

Dabei ist insbesondere der Einsatz von Iminodisuccinat, insbesondere das Tetra-Natriumsalz mit der INCI Tetrasodium Iminodisuccinate erfindungsgemäß bevorzugt. Diese Verbindung kann erfindungsgemäß vorteilhaft in einer Konzentration von 0,1 bis 0,75 Gewichts-%, bezogen auf das Gesamtgewicht der Zubereitung, eingesetzt werden.

Erfindungsgemäß vorteilhafte Ausführungsformen der vorliegenden Erfindung sind auch dadurch gekennzeichnet, dass die kosmetische Zubereitung eine oder mehrere Verbindungen aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride), enthält.

Es ist erfindungsgemäß vorteilhaft, wenn die erfindungsgemäße Zubereitung in Form einer Emulsion oder Dispersion, bevorzugt in Form einer Emulsion und besonders bevorzugt in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate , Natriumstearylglutamat, enthält.

Diese erfindungsgemäß vorteilhaften O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Es ist erfindungsgemäß von Vorteil, wenn die kosmetische Zubereitung eine oder mehrere Polysaccharide enthält. So ist eine erfindungsgemäß bevorzugte Ausführungsform der vorliegenden Erfindung dadurch gekennzeichnet, dass die verwendeten Polysaccharide ausgewählt werden aus der Gruppe der Gumen wie Sclerotium Gum und Cellulose Gum.

Erfindungsgemäß besonders bevorzugt ist der Einsatz von Cellulose Gum. Diese Verbindung kann erfindungsgemäß vorteilhaft in einer Konzentration von 0,1 bis 1,0 Gewichts-%, bezogen auf das Gesamtgewicht der Zubereitung, eingesetzt werden.

Erfindungsgemäß vorteilhafte Ausführungsformen der vorliegenden Erfindung sind dadurch gekennzeichnet, dass die kosmetische Zubereitung eine oder mehrere Polymere aus der Gruppe der Verbindungen Polyvinylalkohol und 1-Ethenyl-pyrrolidin-2-on Polymer mit 1-Triaconten (INCI Triacontanyl PVP, CAS 136445-69-7), enthält.

Eine erfindungsgemäß besonders bevorzugte Ausführungsform der vorliegenden Erfindung ist dadurch gekennzeichnet, dass die kosmetische Zubereitung eine Kombination aus 2-ethylhexyl 2-cyano-3,3-diphenyl-2-propenoat (INCI: Octocrylen) und/oder 2-ethylhexyl 2-hydroxybenzoat (INCI: Ethylhexyl Salicylate), 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine), Iminodisuccinat und Cellulose gum enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Es ist erfindungsgemäß vorteilhaft, wenn die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält. Erfindungsgemäß bevorzugt ist dabei der Einsatz von 1,2-Hexandiol.

Es ist erfindungsgemäß vorteilhaft, wenn die erfindungsgemäße Zubereitung Ethanol, Phenoxyethanol und/oder Ethylhexylglycerin enthält.

Erfindungsgemäß vorteilhafte Ausführungsformen der vorliegenden Erfindung sind dadurch gekennzeichnet dass die Zubereitung frei ist von Parabenen, Methylisothiazolinon, Chlormethylisothiazolinon und DMDM-Hydantoin.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Ansonsten kann die erfindungsgemäße Zubereitung wie eine übliche kosmetische Zubereitung zusammengesetzt sein.

### Vergleichsversuch/Ausführungsbeispiel

Mit dem folgenden Versuch konnte der erfindungsgemäße Effekt beispielhaft belegt werden:

### Messung der Auswaschbarkeit

Es wurden die zwei Sonnenschutzemulsionen (Rezeptur 1 und Rezeptur 2) hinsichtlich der Bildung von gelben Flecken auf Baumwolltextilien über einen in vitro Auftragungs-/ Waschzyklus untersucht. Es wurden dabei weiße vorgewaschene Baumwollmonitore (100% Baumwolle) verwendet. Dazu wurden je 3 mg/cm2 der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet. Nach der Trocknung erfolgte eine farbmetrische Charakterisierung der entstandenen Initial-Verfleckung durch Messung des Gelbgrades mit dem Farbmessgerät DATACOLOR 800 (Datacolor International).
Messgeometrie: d/8°, SCE (Glanzkomponente ausgeschlossen)
Lichtart/Beobachter: D65/10°(entsprechend mittleres Tageslicht)
UV-Filterung: an D65 angepasst, Ganz/Griesser Methode
Messöffnung: LAV (30 mm Durchmesser)
Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen in einer Waschmaschine) (60°C, 2h, Ariel Compact Pulverwaschmittel ,saubere Beiladung).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät DATACOLOR 800 (Datacolor International).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik -Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

### Folgende Rezepturen wurden für die Waschversuche verwendet:

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen

| **Rohstoff [INCI]** | **Rezeptur 1** | **Rezeptur 2** |
|---|---|---|
| Terephthalylidene Dicamphor Sulfonic Acid | - | 2,00 |
| Butylene Glycol Dicaprylate/Dicaprate | 4,50 | 4,50 |
| Dimethicone | 0,90 | 0,90 |
| Glyceryl Stearate | 1,00 | 1,00 |
| Sodium Stearoyl Glutamate | 0,40 | 0,40 |
| Glycerin+Aqua | 7,50 | 7,50 |
| Caprylyl Glycol | 0,25 | 0,25 |
| Aqua+Sodium Hydroxide | 0,25 | 0,25 |
| Phenoxyethanol | 0,80 | 0,80 |
| Cetearyl Alcohol | 2,00 | 2,00 |
| Ammonium Acryloyldimethyl-taurate/VP Copolymer + Aqua | 0,40 | 0,40 |
| Acrylates/C10-30 Alkyl Acrylate Crosspolymer | 0,25 | 0,25 |
| Aqua+Trisodium EDTA | 1,00 | 1,00 |
| Octocrylene | 4,00 | 4,00 |
| Ethylhexyl Salicylate | 4,00 | 4,00 |
| Aqua | 72,75 | 70,75 |
| | | |
| Gesamt | 100,00 | 100,00 |

| | | |
|---|---|---|
| **Gelbwert dB vor dem Waschen** | **11,41** | **5,34** |
| **Gelbwert dB nach dem Waschen** | **8,06** | **1,78** |

### Beispiele für Rezepturen/Sonnenschutzmittel mit denen das erfindungsgemäße Verfahren und die erfindungsgemäße Verwendung durchgeführt werden können:

Alle Mengenangaben, Anteile und Prozentanteile sind, soweit nicht anders angegeben, auf das Gewicht und die Gesamtmenge bzw. auf das Gesamtgewicht der Zubereitungen bezogen.

### O/W Tagescreme

| **Beispielrezeptur 1** | **Gew.-%** |
|---|---|
| Glycerylstearatcitrat | 2 |
| Glycerylstearat | 2 |
| Cetylstearylalkohol | 1 |
| C12-15 Alkylbenzoat | 4 |
| Dicaprylylether | 4 |
| Ethylhexylsalicylate | 3 |
| Octyl Methoxycinnamat | 3 |
| Butylmethoxydibenzoylmethan (z.B. Parsol^{®} 1789) | 2 |
| Terephthalylidene Dicamphor Sulfonic Acid | 1 |
| Carbomer | 0,3 |
| Phenoxyethanol | 0,5 |
| Octandiol | 0,3 |
| 4-Hydroxyacetophenon | 0.3 |
| Natrium Ascorbylphosphat | 0,2 |
| Glycerin | 5 |
| EDTA | 0,2 |
| Parfüm | 0,2 |
| Natronlauge (pH 7 eingestellt) | q.s. |
| Wasser | ad 100 |

### O/W Tagescreme

| **Beispielrezeptur 2** | **Gew.-%** |
|---|---|
| Polyglyceryl-3 Methylglucosedistearat | 3 |
| Glycerylstearat | 2 |
| Cetylalkohol | 1 |
| Isopropylpalmitat | 2 |
| Dicaprylylcarbonat | 2 |
| Caprylsäure/Caprinsäuretriglyceride | 2 |
| Butylmethoxydibenzoylmethan (z.B. Parsol^{®} 1789) | 1 |
| Octocrylen | 2 |
| Ethylhexylsalicylate | 4 |
| Terephthalyliden Dicampher Sulfonsäure | 1 |
| Gylcerylcaprylate | 0,3 |
| Carrageenan | 0,1 |
| Acrylat/C10-30 Alkyl Acrylat Crosspolymer | 0,3 |
| Kreatin | 0,2 |
| Tocopherylacetat | 0,5 |
| Glycerin | 6 |
| EDTA | 0,2 |
| Phenoxyethanol | 0,7 |
| Benzylalkohol | 0.2 |
| Natronlauge (pH 7 eingestellt) | q.s. |
| Wasser | ad 100 |

### O/W Creme

| **Beispielrezeptur 3** | **Gew.-%** |
|---|---|
| Cetearylglucosid | 2 |
| Glycerylstearat | 2 |
| Cetylstearylalkohol | 2 |
| Isopropylpalmitat | 2 |
| Dicaprylylether | 2 |
| C12-15 Alkylbenzoat | 2 |
| 2,4-Bis-{[4-(2-Ethyl-hexyloxy)-2-hydroxy]-phenyl}-6-(4-methoxyphenyl)-1,3,5-triazin (Tinosorb^{®} S) | 2 |
| Sodium Phenylbenzimidazol sulfonate | 0,5 |
| Terephthalyliden Dicampher Sulfonsäure | 0,5 |
| Ethylhexylsalicylate | 5 |
| Acrylat/C10-30 Alkyl Acrylat Crosspolymer | 0,3 |
| Xanthan Gum | 0,2 |
| Glycerin | 7 |
| Tocopherylacetate | 0,5 |
| Panthenol | 0,5 |
| EDTA | 0,2 |
| Ethanol | 3 |
| Ethylhexylglycerin | 0,5 |
| Benzylalkohol | 0,2 |
| Natronlauge (pH 7 eingestellt) | q.s. |
| Wasser | ad 100 |

### Sun Lotion SPF 50

| **Beispielrezeptur 4** | **Gew.-%** |
|---|---|
| Terephthalyliden Dicampher Sulfonsäure | 2 |
| Tocopheryl Acetate | 0,1 |
| Hydroxyacetophenone | 0,4 |
| Panthenol + Aqua | 1,4 |
| Ethylhexylglycerin | 0,3 |
| C12-15 Alkyl Benzoate | 4 |
| Myristyl Myristate | 1 |
| Hydrogenated Coco-Glycerides | 1 |
| Glyceryl Stearate Citrate | 2 |
| VP/Hexadecene Copolymer | 0,5 |
| Parfum | 0,4 |
| Glycerin + Aqua | 5 |
| Aqua + Sodium Hydroxide | 0,05 |
| Cetyl Alcohol | 0,3 |
| Stearyl Alcohol | 0,5 |
| Acrylates/C10-30 Alkyl Acrylate Crosspolymer | 0,1 |
| Xanthan Gum | 0,3 |
| Alcohol Denat. + Aqua | 5 |
| Aqua + Trisodium EDTA | 1 |
| Homosalate | 9 |
| Octocrylene | 9 |
| Ethylhexyl Salicylate | 4,5 |
| Butyl Methoxydibenzoylmethane | 4 |
| Titanium Dioxide (nano) + Silica + Dimethicone | 3 |
| Aqua | ad 100 |

### Sun Lotion SPF 50+

| **Beispielrezeptur 5** | **Gew.-%** |
|---|---|
| Terephthalyliden Dicampher Sulfonsäure | 3 |
| Tocopheryl Acetate | 0,2 |
| Hydroxyacetophenone | 0,4 |
| Panthenol + Aqua | 1,4 |
| Ethylhexylglycerin | 0,3 |
| C12-15 Alkyl Benzoate | 2 |
| Butylene Glycol Dicaprylate/Dicaprate | 2 |
| C18-38 Alkyl Hydroxystearoyl Stearate | 1 |
| Glyceryl Stearate | 1 |
| Sodium Stearoyl Glutamate | 0,3 |
| Silica Dimethyl Silylate | 1 |
| Triacontanyl PVP | 1 |
| Parfum | 0,4 |
| Glycerin + Aqua | 8 |
| Aqua + Sodium Hydroxide | 0,33 |
| Stearyl Alcohol | 1,5 |
| Xanthan Gum | 0,4 |
| Acrylates/C10-30 Alkyl Acrylate Crosspolymer | 0,15 |
| Alcohol Denat. + Aqua | 5 |
| Aqua + Trisodium EDTA | 1 |
| Homosalate | 9 |
| Ethylhexyl Salicylate | 5 |
| Butyl Methoxydibenzoylmethane | 4,5 |
| Titanium Dioxide (nano) + Silica + Dimethicone | 2 |
| Bis-Ethylhexyloxyphenol Methoxyphenyl Triazine | 4 |
| Ethylhexyl Triazone | 3 |
| Phenylbenzimidazole Sulfonic Acid | 1 |
| Aqua | ad 100 |

## Patentansprüche

1. Verwendung von [(3*Z*)-3-[[4-[(*Z*)-[7,7-Dimethyl-2-oxo-1-(sulfomethyl)-3-bicyclo[2.2.1]heptanyliden]methyl]phenyl]methyliden]-7,7-dimethyl-2-oxo-1-bicyclo[2.2.1]heptanyl]methansulfonsäure (INCI: Terephthalylidene dicamphor sulfonic acid) und/oder dessen Natriumsalz in UV-Lichtschutzfilter enthaltenden kosmetischen Zubereitungen, wobei die kosmetische Zubereitung als UV-Filter 2-Ethylhexyl 2-cyano-3,3-diphenyl-2-propenoat (INCI: Octocrylen) und/oder 2-Ethylhexyl 2-hydroxybenzoat (INCI: Ethylhexyl Salicylate) enthält, zur Erleichterung der Auswaschbarkeit der UV-Lichtschutzfilter 2-Ethylhexyl 2-cyano-3,3-diphenyl-2-propenoat (INCI: Octocrylen) und/oder 2-Ethylhexyl 2-hydroxybenzoat (INCI: Ethylhexyl Salicylate) aus mit den Zubereitungen kontaminierten Textilien.

2. Verwendung von [(3*Z*)-3-[[4-[(*Z*)-[7,7-Dimethyl-2-oxo-1-(sulfomethyl)-3-bicyclo[2.2.1]heptanyliden]methyl]phenyl]methyliden]-7,7-dimethyl-2-oxo-1-bicyclo[2.2.1]heptanyl]methansulfonsäure (INCI: Terephthalylidene dicamphor sulfonic acid) und/oder dessen Natriumsalz in UV-Lichtschutzfilter enthaltenden kosmetischen Zubereitungen, wobei die kosmetische Zubereitung als UV-Filter 2-Ethylhexyl 2-cyano-3,3-diphenyl-2-propenoat (INCI: Octocrylen) und/oder 2-Ethylhexyl 2-hydroxybenzoat (INCI: Ethylhexyl Salicylate) enthält, zur Reduzierung der durch von 2-Ethylhexyl 2-cyano-3,3-diphenyl-2-propenoat (INCI: Octocrylen) und 2-Ethylhexyl 2-hydroxybenzoat (INCI: Ethylhexyl Salicylate) hervorgerufenen Textilverfleckung.

3. Verwendung nach einem der vorhergehenden Ansprüche, **dadurch gekennzeichnet, dass** die kosmetische Zubereitung [(3*Z*)-3-[[4-[(*Z*)-[7,7-Dimethyl-2-oxo-1-(sulfomethyl)-3-bicyclo[2.2.1]heptanyliden]methyl]phenyl]methyliden]-7,7-dimethyl-2-oxo-1-bicyclo[2.2.1]heptanyl]methanesulfonsäure (INCI: Terephthalylidene dicamphor sulfonic acid) in einer Konzentration von 0,1 bis 10 Gewichts-%, bezogen auf das Gesamtgewicht der Zubereitung, enthält.

4. Verwendung nach einem der vorhergehenden Ansprüche, **dadurch gekennzeichnet, dass** die kosmetische Zubereitung 2-Ethylhexyl 2-hydroxybenzoat (INCI: Ethylhexyl Salicylate) in einer Konzentration von 0,5 bis 5,0 Gewichts-%, bezogen auf das Gesamtgewicht der Zubereitung, enthält.

5. Verwendung nach einem der vorhergehenden Ansprüche, **dadurch gekennzeichnet, dass** die kosmetische Zubereitung eine oder mehrere Verbindungen gewählt aus der Gruppe 4-(tert.-Butyl)-4'-methoxydibenzoylmethan (INCI Butyl Methoxydibenzoylmethane), (2-[-4-(Diethylamino)-2-hydroxybenzoyl] Benzoesäurehexylester (INCI: Diethylamino Hydroxybenzoyl Hexyl Benzoate) und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

6. Verwendung nach einem der vorhergehenden Ansprüche, **dadurch gekennzeichnet, dass** die kosmetische Zubereitung eine oder mehrere Verbindungen gewählt aus der Gruppe 2,4,6-Tris-[anilino-(p-carbo-2'-ethyl-1'-hexyloxy)]-1,3,5-triazin (INCI: Ethylhexyl Triazone), 3,3,5-Trimethylcyclohexyl 2-hydroxybenzoat (INCI: Homosalate), Titandioxid und 2-Phenylbenzimidazol-5-sulfonsäuresalzen enthält.

7. Verwendung nach einem der vorhergehenden Ansprüche, **dadurch gekennzeichnet, dass** die kosmetische Zubereitung eine oder mehrere der Verbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat
- Natriumpolyphosphat
- Tetranatriumpyrophosphat
- Hydroxamsäure
- Polygalacturonsäure
- Bernsteinsäure
- Ameisensäure
- Äpfelsäure
und/oder deren Alkalisalze und/oder
1-Hydroxy-4-methyl-6-(2,4,4-trimethylpentyl)-2(1H)-pyridon (Pirocton) oder dessen Monoethanolamin-Salz
enthält.

8. Verwendung nach einem der vorhergehenden Ansprüche, **dadurch gekennzeichnet, dass** die kosmetische Zubereitung eine oder mehrere Verbindungen aus der Gruppe C12-15 Alkylbenzoate (INCI: C12-15 Alkyl Benzoate), 2-Octyldodecan-1-ol (INCI: Octyldodecanol), Capryl/Caprinsäure Triglyceride (INCI: Caprylic/Capric Triglyceride), enthält.

9. Verwendung nach einem der vorhergehenden Ansprüche, **dadurch gekennzeichnet, dass** die kosmetische Zubereitung eine oder mehrere Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate , Natriumstearylglutamat, enthält.

10. Verwendung nach einem der vorhergehenden Ansprüche, **dadurch gekennzeichnet, dass** die kosmetische Zubereitung eine oder mehrere Polysaccharide enthält.

11. Verwendung nach einem der vorhergehenden Ansprüche, **dadurch gekennzeichnet, dass** die kosmetische Zubereitung eine oder mehrere Polymere aus der Gruppe der Verbindungen Polyvinylalkohol und 1-Ethenyl-pyrrolidin-2-on Polymer mit 1-Triaconten (INCI Triacontanyl PVP, CAS 136445-69-7), enthält.

12. Verwendung nach einem der vorhergehenden Ansprüche, **dadurch gekennzeichnet, dass** die kosmetische Zubereitung eine Kombination aus 2-Ethylhexyl 2-cyano-3,3-diphenyl-2-propenoat (INCI: Octocrylen) und/oder 2-Ethylhexyl 2-hydroxybenzoat (INCI: Ethylhexyl Salicylate), 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine), Iminodisuccinat und Cellulose gum enthält.

## Claims

1. Use of [(3*Z*)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI: Terephthalylidene dicamphor sulfonic acid) and/or the sodium salt thereof in cosmetic preparations comprising UV light protection filters, wherein the cosmetic preparation comprises, as UV filter, 2-ethylhexyl 2-cyano-3,3-diphenyl-2-propenoate (INCI: Octocrylene) and/or 2-ethylhexyl 2-hydroxybenzoate (INCI: Ethylhexyl Salicylate), to facilitate the ability of the UV light protection filters 2-ethylhexyl 2-cyano-3,3-diphenyl-2-propenoate (INCI: Octocrylene) and/or 2-ethylhexyl 2-hydroxybenzoate (INCI: Ethylhexyl Salicylate) to be washed out of textiles contaminated with the preparations.

2. Use of [(3*Z*)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI: Terephthalylidene dicamphor sulfonic acid) and/or the sodium salt thereof in cosmetic preparations comprising UV light protection filters, wherein the cosmetic preparation comprises, as UV filter, 2-ethylhexyl 2-cyano-3,3-diphenyl-2-propenoate (INCI: Octocrylene) and/or 2-ethylhexyl 2-hydroxybenzoate (INCI: Ethylhexyl Salicylate), for reducing the textile staining caused by 2-ethylhexyl 2-cyano-3,3-diphenyl-2-propenoate (INCI: Octocrylene) and 2-ethylhexyl 2-hydroxybenzoate (INCI: Ethylhexyl Salicylate).

3. Use according to any of the preceding claims, **characterized in that** the cosmetic preparation contains [(3*Z*)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI: Terephthalylidene dicamphor sulfonic acid) in a concentration of 0.1% to 10% by weight, based on the total weight of the preparation.

4. Use according to any of the preceding claims, **characterized in that** the cosmetic preparation comprises 2-ethylhexyl 2-hydroxybenzoate (INCI: Ethylhexyl Salicylate) in a concentration of 0.5% to 5.0% by weight, based on the total weight of the preparation.

5. Use according to any of the preceding claims, **characterized in that** the cosmetic preparation comprises one or more compounds selected from the group of 4-(tert-butyl)-4'-methoxydibenzoylmethane (INCI Butyl Methoxydibenzoylmethane), hexyl (2-[4-(diethylamino)-2-hydroxybenzoyl]benzoate (INCI: Diethylamino Hydroxybenzoyl Hexyl Benzoate) and/or 2,4-bis{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine).

6. Use according to any of the preceding claims, **characterized in that** the cosmetic preparation comprises one or more compounds selected from the group of 2,4,6-tris[anilino(p-carbo-2'-ethyl-1'-hexyloxy)]-1,3,5-triazine (INCI: Ethylhexyl Triazone), 3,3,5-trimethylcyclohexyl 2-hydroxybenzoate (INCI: Homosalate), titanium dioxide and 2-phenylbenzimidazole-5-sulfonic acid salts.

7. Use according to any of the preceding claims, **characterized in that** the cosmetic preparation comprises one or more of the compounds selected from the group of
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- iminodisuccinate
- sodium polyphosphate
- tetrasodium pyrophosphate
- hydroxamic acid
- polygalacturonic acid
- succinic acid
- formic acid
- malic acid
and/or their alkali metal salts and/or
1-hydroxy-4-methyl-6-(2,4,4-trimethylpentyl)-2(1H)-pyridone (Piroctone) or its monoethanolamine salt.

8. Use according to any of the preceding claims, **characterized in that** the cosmetic preparation comprises one or more compounds from the group of C12-15 alkyl benzoates (INCI: C12-15 Alkyl Benzoate), 2-octyldodecan-1-ol (INCI: Octyldodecanol), caprylic/capric acid triglycerides (INCI: Caprylic/Capric Triglyceride).

9. Use according to any of the preceding claims, **characterized in that** the cosmetic preparation comprises one or more emulsifiers selected from the group of the compounds glyceryl stearate citrate, glyceryl stearate (self-emulsifying),
stearic acid, stearate salts, polyglyceryl-3 methylglycose distearate, sodium cetearyl sulfate, potassium cetyl phosphate, polyglyceryl-10 stearate, sodium stearyl glutamate.

10. Use according to any of the preceding claims, **characterized in that** the cosmetic preparation comprises one or more polysaccharides.

11. Use according to any of the preceding claims, **characterized in that** the cosmetic preparation comprises one or more polymers from the group of the compounds polyvinyl alcohol and 1-ethenylpyrrolidin-2-one polymer with 1-triacontene (INCI Triacontanyl PVP, CAS 136445-69-7).

12. Use according to any of the preceding claims, **characterized in that** the cosmetic preparation comprises a combination of 2-ethylhexyl 2-cyano-3,3-diphenyl-2-propenoate (INCI: Octocrylene) and/or 2-ethylhexyl 2-hydroxybenzoate (INCI: Ethylhexyl Salicylate), 2,4-bis{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine), iminodisuccinate and cellulose gum.

## Revendications

1. Utilisation de l'acide [(3Z)-3-[[4-[(Z)-[7,7-diméthyl-2-oxo-1 -(sulfométhyl)-3-bicyclo[2.2.1]heptanylidène]méthyl]phényl]méthylidène]-7,7-diméthyl-2-oxo-1-bicyclo[2.2.1]heptanyl]méthanesulfonique (INCI : Terephthalylidene dicamphor sulfonic acid) et/ou de son sel sodique dans des préparations cosmétiques contenant des filtres de protection contre la lumière UV, la préparation cosmétique contenant, en tant que filtre UV, du 2-cyano-3,3-diphényl-2-propénoate de 2-éthylhexyle (INCI : Octocrylen) et/ou du 2-hydroxybenzoate de 2-éthylhexyle (INCI : Ethylhexyl Salicylate) afin de faciliter la rinçabilité des filtres de protection contre la lumière UV 2-cyano-3,3-diphényl-2-propénoate de 2-éthylhexyle (INCI : Octocrylen) et/ou 2-hydroxybenzoate de 2-éthylhexyle (INCI : Ethylhexyl Salicylate) à partir de textiles contaminés par les préparations.

2. Utilisation de l'acide [(3Z)-3-[[4-[(Z)-[7,7-diméthyl-2-oxo-1-(sulfométhyl)-3-bicyclo[2.2.1]heptanylidène]méthyl]phényl]méthylidène]-7,7-diméthyl-2-oxo-1-bicyclo[2.2.1]heptanyl]méthanesulfonique (INCI : Terephthalylidene dicamphor sulfonic acid) et/ou de son sel sodique dans des préparations cosmétiques contenant des filtres de protection contre la lumière UV, la préparation cosmétique contenant, en tant que filtre UV, du 2-cyano-3,3-diphényl-2-propénoate de 2-éthylhexyle (INCI : Octocrylen) et/ou du 2-hydroxybenzoate de 2-éthylhexyle (INCI : Ethylhexyl Salicylate) afin de réduire les taches sur les textiles provoquées par le 2-cyano-3,3-diphényl-2-propénoate de 2-éthylhexyle (INCI : Octocrylen) et/ou le 2-hydroxybenzoate de 2-éthylhexyle (INCI : Ethylhexyl Salicylate).

3. Utilisation selon l'une des revendications précédentes, **caractérisée en ce que** la préparation cosmétique contient l'acide [(3Z)-3-[[4-[(Z)-[7,7-diméthyl-2-oxo-1-(sulfométhyl)-3-bicyclo[2.2.1]heptanylidène]méthyl]phényl]méthylidène]-7,7-diméthyl-2-oxo-1-bicyclo[2.2.1]heptanyl]méthanesulfonique (INCI : Terephthalylidene dicamphor sulfonic acid) en une concentration de 0,1 à 10% en poids, par rapport au poids total de la préparation.

4. Utilisation selon l'une des revendications précédentes, **caractérisée en ce que** la préparation cosmétique contient le 2-hydroxybenzoate de 2-éthylhexyle (INCI : Ethylhexyl Salicylate) en une concentration de 0,5 à 5,0% en poids, par rapport au poids total de la préparation.

5. Utilisation selon l'une des revendications précédentes, **caractérisée en ce que** la préparation cosmétique contient un ou plusieurs composés, choisis dans le groupe 4-(tert-butyl)-4'-méthoxydibenzoylméthane (INCI : Butyl Methoxydibenzoylmethane), ester hexylique de l'acide 2-[-4-(diéthylamino)-2-hydroxybenzoyl]benzoïque (INCI : Diethylamino Hydroxybenzoyl Hexyl Benzoate) et/ou 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

6. Utilisation selon l'une des revendications précédentes, **caractérisée en ce que** la préparation cosmétique contient un ou plusieurs composés choisis dans le groupe 2,4,6-tris-[anilino-(p-carbo-2'-éthyl-1'-hexyloxy)]-1,3,5-triazine (INCI : Ethylhexyl Triazone), 2-hydroxybenzoate de 3,3,5-triméthylcyclohexyle (INCI : Homosalate), dioxyde de titane et sels de l'acide 2-phénylbenzimidazole-5-sulfonique.

7. Utilisation selon l'une des revendications précédentes, **caractérisée en ce que** la préparation cosmétique contient un ou plusieurs des composés du groupe
- acide 1-hydroxyéthane-1,1-diphosphonique/HEDP
- acide aminotriméthylènephosphonique/ATMP
- acide diéthylène-triamine-penta(méthylènephosphonique)/DTPMP
- acide éthylène-diamine-tétraméthylènephosphonique/EDTMP
- acide phosphonobutanetricarboxylique/PBTC
- iminodisuccinate
- polyphosphate de sodium
- pyrophosphate de tétrasodium
- acide hydroxamique
- poly(acide galacturonique)
- acide succinique
- acide formique
- acide malique
et/ou leurs sels alcalins et/ou
1-hydroxy-4-méthyl-6-(2,4,4-triméthylpentyl)-2(1 H)-pyridone (Pirocton) ou son sel de monoéthanolamine

8. Utilisation selon l'une des revendications précédentes, **caractérisée en ce que** la préparation cosmétique contient un ou plusieurs composés du groupe benzoate de C12-15-alkye (INCI : C12-15 Alkyl Benzoate), 2-octyldodécan-1-ol (INCI : Octyldodecanol), triglycéride d'acide caprylique/caprique (INCI : Caprylic/Capric Triglyceride).

9. Utilisation selon l'une des revendications précédentes, **caractérisée en ce que** la préparation cosmétique contient un ou plusieurs émulsifiants choisis dans le groupe de composés stéarate-citrate de glycéryle, stéarate de glycéryle (auto-émulsifiant),
acide stéarique, sels de stéarate, 3-méthylglycosedistéarat de polyglycéryle, cétéarylsulfate de sodium, cétylphosphate de potassium, stéarate de polyglycéryle-10, stéarylglutamate de sodium.

10. Utilisation selon l'une des revendications précédentes, **caractérisée en ce que** la préparation cosmétique contient un ou plusieurs polysaccharides.

11. Utilisation selon l'une des revendications précédentes, **caractérisée en ce que** la préparation cosmétique contient un ou plusieurs polymères du groupe de composés poly(alcool vinylique) et polymère de 1-éthényl-pyrrolidin-2-one avec du 1-triacontène (INCI : Triacontanyl PVP, CAS 136445-69-7).

12. Utilisation selon l'une des revendications précédentes, **caractérisée en ce que** la préparation cosmétique contient une combinaison de 2-cyano-3,3-diphényl-2-propénoate de 2-éthylhexyle (INCI : Octocrylen) et/ou de 2-hydroxybenzoate de 2-éthylhexyle (INCI : Ethylhexyl Salicylate), de 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine), d'iminodisuccinate et de gomme de cellulose.
